(19) Europäisches Patentamt
European Patent Office
Office européen des brevets

(11) Publication number: 0 461 520 A2

(12) # EUROPEAN PATENT APPLICATION

(21) Application number: 91109086.8

(22) Date of filing: 04.06.91

(51) Int. Cl.5: C07H 19/02, A61K 31/70

(30) Priority: 12.06.90 JP 151553/90

(43) Date of publication of application: 18.12.91 Bulletin 91/51

(84) Designated Contracting States: DE FR GB IT

(71) Applicant: Nippon Kayaku Kabushiki Kaisha
11-2, Fujimi-cho 1 chome Chiyoda-ku
Tokyo(JP)

(72) Inventor: Izawa, Takao
1104-17, Kounosu
Koga-shi, Ibaraki-ken(JP)
Inventor: Chikui, Yukio
3-33-5, Shimo
Kita-ku,Tokyo(JP)
Inventor: Imaizumi, Yoshihiro
3-17-10, Shimo
Kita-ku, Tokyo(JP)
Inventor: Iwasawa, Haruo
384, Kitahirasawa, Hidaka-machi
Iruma-gun, Saitama-ken(JP)
Inventor: Ogawa, Yutaka
7-1-49, Nishi, Shiraoka-machi
Minamisaitama-gun, Saitama-ken(JP)

(74) Representative: Türk, Gille, Hrabal
Brucknerstrasse 20
W-4000 Düsseldorf 13(DE)

(54) ## Oxetanocin G anhydride crystals and process for producing the same.

(57) The present invention relates to stable crystals of oxetanocin G anhydride which are produced by dissolving oxetanooin G hydrate or a salt thereof in water and crystallizing out oxetanocin G at a high temperature to thereby give oxetanocin G anhydride crystals having no hygroscopicity and a high heat stability.

Fig. 1

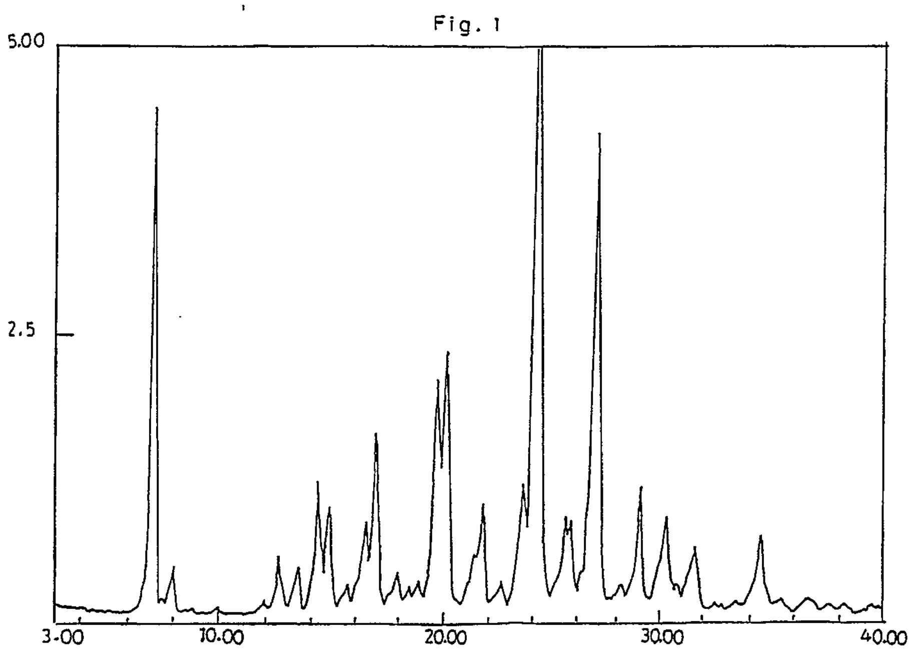




Rank Xerox (UK) Business Services

Background of the Invention:

Oxetanocin G, which has an antiviral activity, is disclosed as a powder in EP-A3-0291917 and as a monohydrate in the Journal of Antibiotics, 40 (12), 1788 - 1790 (1987).

However these oxetanocin G powder and monohydrate (both of which will be referred to as oxetanocin G hydrate as a whole hereinafter) have some disadvantages, from the viewpoint of the production of pharmaceutical preparations, that they have a hygroscopicity and suffer from variations in moisture content (ranging from approximately 3.0% to approximately 6.5%) since they would liberate water of crystallization under some drying conditions; that they have a somewhat poor heat stability; and that the crystalline form thereof would be easily changed by, for example, physical grinding.

Under these circumstances, the present inventors have conducted extensive studies in order to solve these problems. As a result, they have found out that oxetanocin G anhydride crystals which have no hygroscopicity and a high heat stability and the crystalline form of which is never changed by, for example, grinding can be obtained by dissolving oxetanocin G hydrate or a salt thereof in water. (further neutralizing the resulting solution with an appropriate acid, when a salt is used), and then crystallizing oxetanocin G out of the solution at a high temperature, thus completed the present invention.

Summary of the Invention:

It is an object of the present invention to provide oxetanocin G anhydride crystals. The anhydride crystals of the present invention show the following major absorption peaks in an infrared absorption spectrum:

IR: 3300, 3170, 1722, 1635, 1580, 1383, 1023 and 973 $cm^{-1}$.

These anhydride crystals can be produced by crystallizing oxetanocin G out of an aqueous solution of oxetanocin G under heating.

The anhydride crystals of the present invention, which is non-hygroscopic and has no water of crystallization, remains stable without showing any change in weight even at a high humidity or at a high temperature. Therefore they can be easily treated in the production of a pharmaceutical preparation.

Brief Description of the Drawing:

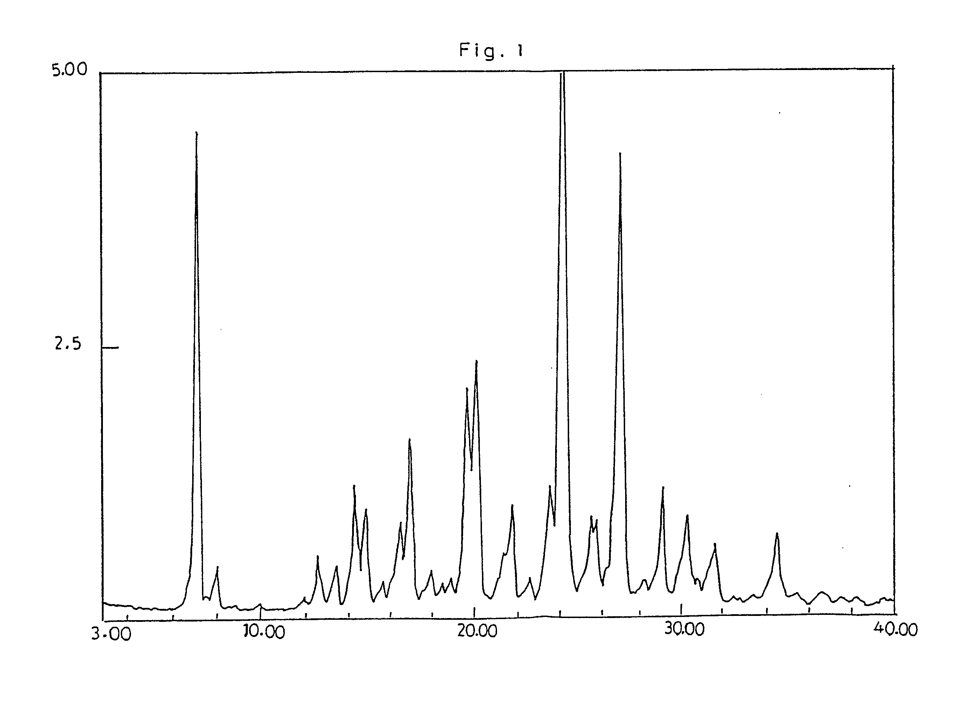

Fig. 1 is an X-ray diffraction pattern of the anhydride crystals of the present invention.

Detailed Description of the Invention:

A known oxetanocin G hydrate may be usually obtained by treating 2-aminooxetanocin A represented by the following formula:

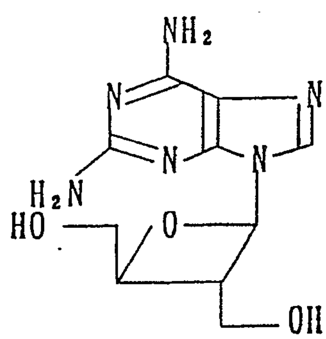

with adenosine deaminase followed by concentration to dryness (refer to EP-A3-0291917).

The oxetanocin G hydrate thus obtained is then treated in a conventional manner, namely, by dissolving in water under heating, cooling to approximately 5° C, recrystallizing, filtering, and drying at room temperature under reduced pressure. Thus monohydrate crystals having a moisture content of approximately 6% are obtained. A salt of oxetanocin G may be prepared by using an acid (for example, hydrochloric acid, sulfuric acid or phosphoric acid) or a base. In the present invention, a salt of oxetanocin G with a base is preferable. The oxetanocin G salt may be produced in a conventional manner, namely, by

suspending the aforesaid hydrate in water, adding approximately one equivalent of a base (for example, an inorganic base such as sodium hydroxide or potassium hydroxide or organic base such as ammonia) for dissolving it in water, and adding an appropriate solvent to thereby crystallize out or concentrating to dryness.

The aqueous solution of oxetanocin G to be used in the present invention may be usually obtained by suspending oxetanocin G hydrate obtained by the aforesaid known method or a salt thereof in approximately 10 times (v/w) or more, preferably approximately 15 to approximately 50 times and more preferably approximately 20 to 30 times, as much as water and dissolving it under heating preferably to 60 ° C or above and more preferably to a temperature from approximately 70 ° C to approximately 100 ° C. When a salt is used, the solution thus heated is neutralized by adding an appropriate acid, for example, an inorganic acid such as hydrochloric acid, sulfuric acid or phosphoric acid or an organic acid such as acetic acid or oxalic acid. When free oxetanocin G is used, an appropriate base may be added to water so as to facilitate the dissolution of oxetanocin G, and then the oxetanocin G is dissolved therein by heating followed by neuralization in the same manner as the one employed in the case of a salt.

It is sometimes possible to use a solution which is obtained by concentrating a column eluate of oxetanocin G synthesized from 2-aminooxetanocin A in such a manner as to give an oxetanocin G concentration of approximately 2% (w/v; the same will apply hereinafter unless otherwise noted) or above and then dissolving it by heating preferably to 60 ° C or above and more preferably to a temperature from approximately 70 ° C to approximately 100 ° C.

The oxetanocin G concentration in such a heated aqueous solution may be approximately 2% or above, preferably approximately 3% or above and more preferably from approximately 4% to approximately 10%. The aqueous solution may further contain organic solvent(s) miscible with water so long as they exert no adverse effects on the anhydride crystals.

The oxetanocin G anhydride crystals of the present invention can be obtained from the aforesaid solution of oxetanocin G by maintaining said solution at a temperature of approximately 40 ° C or above, preferably from approximately 50 ° C to approximately 70 ° C and more preferably from approximately 60 ° C to approximately 65 ° C. During this period, crystals of oxetanocin G anhydride are formed and crystallized out.

After maintaining the solution at the aforesaid temperature until most of the oxetanocin G is crystallized out, the solution is cooled and thus the residual anhydride crystals formed in the solution are crystallized out.

Although the period of time for maintaining said temperature would vary depending on, for example, concentration, it may be usually approximately 0.2 hour or longer, preferably from approximately 0.5 hour to approximately 3 hours and more preferably from approximately 1 hour to approximately 2 hours.

An oxetanocin G hydrate obtained by a conventional method is unsuitable for the formulation into a pharmaceutical preparation, because the moisture content thereof would vary from approximately 3 to approximately 6.5% depending on drying conditions, it is highly hygroscopic in the atmosphere and the crystalline form thereof is easily changed by, for example, grinding in a mortar. In contrast, the crystals obtained by the present invention, which have no hygroscopicity and a high heat stability and suffer from no change in the crystalline form even by grinding, are highly suitable for the formulation.

Test Example

(1) Change in moisture content with drying conditions

Crystals of oxetanocin G hydrate recrystallized from water and the anhydride crystals of the present invention were dried under the conditions as specified below. The following table shows the results.

| Drying conditions | Moisture content (w/w %) | |
|---|---|---|
| | Invention (anhydride crystals) | Control (hydrate) |
| Drying at room temp. under reduced pressure (3.0 mmHg) for 6 hrs | 0.4 | 6.4 |
| Drying at 40 to 50°C under reduced pressure (3.0 mmHg) for 6 hrs | 0.3 | 3.6 |

(2) Hygroscopicity test

The oxetanocin G anhydride crystals obtained in the present invention and a known oxetanocin G hydrate were stored under a relative humidity of 75% at 25°C for 48 hours. As a result, each sample showed the following weight gain (w/w %).

Weight gain after 48 hours

| Storage period | 48 hr |
|---|---|
| Control (hydrate) (initial moisture content: 3.6%) | 6.5% |
| Anhydride crystals of invention (initial moisture content: 0.3%) | 0.8% |

To further illustrate the present invention, the following Example will be given.

Example

4.01 g of oxetanocin G hydrate (moisture content: 3.0%), 15 ml of 1 N sodium hydroxide and 50 ml of distilled water were fed into a four-necked flask (200 ml). After completely dissolving the crystals, the temperature of the solution was elevated to 70°C. Then 15 ml of 1 N hydrochloric acid was added thereto so as to adjust the pH to 7.1. The crystals thus precipitated were dissolved again and then the temperature of the solution was lowered to 63°C. Then the crystals were crystallized out under stirring for 2 hours. Next, the temperature of the mixture was lowered to 3°C and the crystals were filtered under sucking and then dried under reduced pressure (45°C/2.0 mmHg/10 hours). Thus 3.48 g of oxetanocin G anhydride was obtained (yield: 89.5%).

m.p.: 232°C (decomp.). (determined with a Shimadzu thermo-gravimeter TGA-50).
Specific rotation: $[\alpha]_D^{20}$ - 24.4°(C = 1.0, 0.1 N NaOH).
IR (KBr tablet): 3300, 3170, 1722, 1635, 1580, 1383, 1023 and 973 ($cm^{-1}$).
Moisture content: 0.3 %.
NMR: Agreed with a separately prepared synthetic sample.

X-ray diffraction peaks:

(anhydride crystals of invention)

2θ(°): 7.19, 14.41, 17.02, 19.71, 20.13, 24.27 and 27.17.

## Claims

1. Oxetanocin G anhydride crystals showing the following major absorption peaks in an infrared absorption spectrum:
   IR: 3300, 3170, 1722, 1635, 1580, 1383, 1023 and 973 $cm^{-1}$.

2. A process for producing oxetanocin G anhydride crystals which comprises crystallizing out oxetanocin G from an aqueous solution of oxetanocin G under heating.

3. A process as claimed in Claim 2, wherein the temperature of said aqueous solution, out of which oxetanocin G is to be crystallized, is approximately 40° C or above.

4. A process as claimed in Claim 2, wherein the concentration of oxetanocin G in the heated aqueous solution is approximately 2% or above.

5. A pharmaceutical composition which comprises oxetanocin G anhydride crystals as claimed in Claim 1 or produced by a process as claimed in any of Claims 2 to 4 as an active ingredient together with pharmaceutically acceptable carrier(s) or diluent(s).

Fig. 1